(19) Europäisches Patentamt
European Patent Office
Office européen des brevets

(11) **EP 2 717 939 B1**

(12) **EUROPÄISCHE PATENTSCHRIFT**

(45) Veröffentlichungstag und Bekanntmachung des Hinweises auf die Patenterteilung:
**31.08.2016 Patentblatt 2016/35**

(21) Anmeldenummer: **12727792.9**

(22) Anmeldetag: **06.06.2012**

(51) Int Cl.:
*A61M 1/16* (2006.01)　　　*G01F 1/74* (2006.01)

(86) Internationale Anmeldenummer:
**PCT/EP2012/002417**

(87) Internationale Veröffentlichungsnummer:
**WO 2012/167931 (13.12.2012 Gazette 2012/50)**

(54) **VORRICHTUNG ZUM ÜBERPRÜFEN DER FÖRDERLEISTUNG MINDESTENS EINES FÖRDERMITTELS EINER VORRICHTUNG ZUR EXTRAKORPORALEN BLUTBEHANDLUNG**

DEVICE FOR TESTING THE DELIVERY RATE OF AT LEAST ONE DELIVERY MEANS OF A DEVICE FOR EXTRACORPOREAL BLOOD TREATMENT

DISPOSITIF DE CONTRÔLE DE LA CAPACITÉ DE REFOULEMENT D'AU MOINS UN MOYEN DE TRANSPORT D'UN DISPOSITIF DE TRAITEMENT EXTRACORPOREL DU SANG

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.06.2011 DE 102011106113**
**09.06.2011 US 201161495054 P**

(43) Veröffentlichungstag der Anmeldung:
**16.04.2014 Patentblatt 2014/16**

(73) Patentinhaber: **Fresenius Medical Care Deutschland GmbH**
**61352 Bad Homburg (DE)**

(72) Erfinder: **GAGEL, Alfred**
**96123 Litzendorf (DE)**

(74) Vertreter: **Ziermann, Oliver**
**Fresenius Medical Care AG & Co. KGaA**
**Global Patents & IP**
**Else-Kröner-Strasse 1**
**61352 Bad Homburg (DE)**

(56) Entgegenhaltungen:
**DE-A1- 19 919 572　　US-A1- 2005 069 425**
**US-A1- 2009 012 455**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft eine Vorrichtung zum Überprüfen der Förderleistung mindestens eines Fördermittels für medizinische Flüssigkeiten an Vorrichtungen zur extrakorporalen Blutbehandlung sowie eine Blutbehandlungsvorrichtung.

Es sind verschiedene Arten von Blutbehandlungsvorrichtungen bekannt. Zu den bekannten Blutbehandlungsvorrichtungen zählen beispielsweise die Vorrichtungen zur Hämodialyse, Hämofiltration und Hämodiafiltration. Während der extrakorporalen Blutbehandlung strömt das Blut in einem extrakorporalen Blutkreislauf durch eine Blutbehandlungseinheit. Bei den Vorrichtungen zur Hämodialyse, Hämofiltration und Hämodiafiltration ist die Blutbehandlungseinheit ein Dialysator oder Filter, der vereinfacht ausgedrückt durch eine semipermeable Membran in eine Blutkammer und eine Dialysierflüssigkeitskammer getrennt ist. Während der Blutbehandlung mittels Hämodialyse oder Hämodiafiltration strömt das Blut durch die Blutkammer, während eine Dialysierflüssigkeit durch die Dialysierflüssigkeitskammer strömt.

Dokument US 2009/012455 offenbart eine Art von Blutbehandlungsvorrichtung.

Die Bereitstellung tür die Dialysierflüssigkeit kann durch ein Dialysierflüssibkeitssystem erfolgen, das in die Vorrichtung zur extrakorporalen Blutbehandlung integriert ist. In dem Dialysierflüssigkeitssystem kann Reinwasser z.B. aus einer Umkehrosmose zugeführt werden, dass zunächst entgast und dann mit Flüssigkonzentraten zur Herstellung von frischer Dialysierflüssigkeit vermischt wird. Die Vermischung kann beispielsweise durch Zugabe der Flüssigkonzentrate an getrennten Zugabestellen in die Reinwasserleitung oder durch Zugabe über getrennte Zugabestellen in eine Mischkammer erfolgen. Die frische Dialysierflüssigkeit durchströmt zunächst ein Bilanziersystem und wird danach durch die Dialysierkammer des Dialysators geleitet. Dabei wird die Dialysierflüssigkeit mit Wasser und Inhaltsstoffen des Bluts beladen und wird dadurch zur gebrauchten Dialysierflüssigkeit. Nach dem Verlassen des Dialysators wird die gebrauchte Dialysierflüssigkeit durch das Bilanziersystem geleitet.

Dabei wird eine Differenz zwischen dem Volumen der frischen Dialysierflüssigkeit und der gebrauchten Dialysierflüssigkeit ermittelt.

Die Mischkammer weist zuführende Flüssigkeitsleitungen und abführende Flüssigkeitsleitungen auf. Der Mischkammer wird unvollständig vorgemischtes Gemisch aus entgastem Reinwasser und Flüssigkonzentraten zugeführt. In der Mischkammer erfolgt die vollständige Vermischung. Frische Dialysierflüssigkeit wird aus der Mischkammer durch eine Dialysierflüssigkeitsleitung abgeführt. Die Förderung von Reinwasser, Flüssigkonzentraten und Dialysierflüssigkeit in den Leitungen erfolgt durch Fördermittel, beispielsweise durch Pumpen. Die Entgasung des Reinwassers erfolgt durch Erzeugen eines Unterdrucks mittels einer Entgasungspumpe in die Reinwasserleitung stromaufwärts der Mischkammer. Die Förderung der Flüssigkonzentrate erfolgt durch Dosierpumpen stromaufwärts der Mischkammer. Die Förderung der Dialysierflüssigkeit erfolgt durch eine Dialysierflüssigkeitspumpe in der Dialysierflüssigkeitsleitung. Mit der Dialysierflüssigkeitsleitung vor dem Dialysator und mit der Dialysierflüssigkeitsleitung nach dem Dialysator können weitere Pumpen in flüssigkeitsführender Verbindung stehen, wie beispielsweise die Flusspumpe in der Dialysierflüssigkeitsleitung und eine Ultrafiltrationspumpe.

In Blutbehandlungsmaschinen kann die Dialysierflüssigkeit durch volumetrische Mischung hergestellt werden. Unter volumetrischer Mischung ist zu verstehen, dass mindestens eine Flüssigkeit nach dem Volumen dosiert wird. Beispielsweise können Reinwasser und mindestens ein Flüssigkonzentrat volumetrisch dosiert und entsprechend einer vorgegebenen Rezeptur zu frischer Dialysierflüssigkeit vermischt werden.

Bei der volumetrischen Dosierung ist die Genauigkeit der Förderleistung der Fördermittel von entscheidender Bedeutung. Eine fehlerhafte Dosierung führt zu einer fehlerhaften Zusammensetzung der Dialysierflüssigkeit. Die Förderleistung eines Fördermittels kann sich mit der Zeit ändern. Ursachen für solche Änderungen der Förderleistung eines Fördermittels können beispielsweise Undichtigkeiten, Quellungen von Werkstoffen, Ablagerungen im Inneren des Fördermittels und/oder den Leitungen und Verschleiß sein. Während Undichtigkeiten mit einem automatischen Integritätstest, beispielsweise einem Druckhaltetest auf einfache Weise sicher erkannt werden können, sind die anderen Ursachen, die zu einer größeren oder einer kleineren Förderleistung führen können, nicht ohne Weiteres automatisch erkennbar.

Um die Patientensicherheit nicht zu gefährden, ist eine fehlerhafte Dosierung auszuschließen. Deshalb werden die Fördermittel so robust ausgelegt, dass eine betriebsbedingte Änderung der Förderleistung während ihrer Lebensdauer möglichst klein ist. Dennoch kann sich die Förderleistung eines Fördermittels mit der Zeit ändern.

Die Förderleistung wird im Rahmen regelmäßiger Wartungsintervalle überprüft und bei Bedarf korrigiert. Die Förderleistung kann dabei durch Auslitern ermittelt werden. Solche Arbeiten werden von einem qualifizierten Servicetechniker ausgeführt.

Je öfter die Fördermittel der Blutbehandlungsvorrichtung überprüft werden, desto zuverlässiger kann die Blutbehandlungsvorrichtung arbeiten. Es besteht der Bedarf nach einer regelmäßigen automatischen Überprüfung der Förderleistung. Es besteht ein Bedarf nach einer automatischen Erkennung von Änderungen der Förderleistung eines Fördermittels, beispielsweise im Rahmen eines regelmäßigen automatischen Funktionstests.

Eine Aufgabe der Erfindung ist es, ein gattungsgemäßes Blutbehandlungsgerät derart weiterzubilden, dass das Überprüfen der Förderleistung mindestens eines ersten oder eines zweiten Fördermittels automatisch erfolgen kann, ohne dass der Einsatz eines qualifizierten Servicetechnikers oder eines Anwenders erforderlich ist.

Eine weitere Aufgabe der Erfindung ist es, ein gattungsgemäßes Blutbehandlungsgerät derart weiterzubilden, dass das Erkennen einer Abweichung der Förderleistung mindestens eines ersten oder eines zweiten Fördermittels von dem Sollwertebereich bereits einen Alarm auslöst, ohne dass notwendigerweise ermittelt wird, welches der beiden Fördermittel eine Abweichung der Förderleistung aufweist oder ob beide Fördermittel eine Abweichung der Förderleistung aufweisen.

Eine weitere Aufgabe beinhaltet nicht nur das Erkennen einer Abweichung der Förderleistung mindestens eines ersten oder eines zweiten Fördermittels von dem Sollwertebereich, sondern darüber hinaus auch einen weiteren Schritt zur Ermittlung, welches der beiden Fördermittel eine Abweichung der Förderleistung aufweist oder ob beide Fördermittel eine Abweichung der Förderleistung aufweisen.

Eine weitere Aufgabe der vorliegenden Erfindung ist die Verbesserung der Benutzerfreundlichkeit eines gattungsgemäßen Blutbehandlungsgeräts, so dass der Anwender, beispielsweise der behandelnde Arzt oder die Dialyseschwester, nicht mit der Überprüfung und Korrektur der Förderleistung von Fördermitteln belastet werden und dafür auch keine Ausfallzeiten der Blutbehandlungsmaschine während des Einsatzes eines qualifizierten Servicetechnikers entstehen. Dazu soll eine automatische Korrektur der Förderleistung mindestens des ersten oder des zweiten Fördermittels bei Abweichung dessen Förderleistung von dem Sollwertebereich erfolgen.

Eine weitere Aufgabe der vorliegenden Erfindung ist die Erhöhung der Zuverlässigkeit der Blutbehandlungsvorrichtung.

Eine weitere Aufgabe der vorliegenden Erfindung ist die Erhöhung der Sicherheit der Blutbehandlungsvorrichtung. Je genauer die Fördermittel der Blutbehandlungsvorrichtung fördern, desto sicherer kann die Blutbehandlungsvorrichtung arbeiten.

Die Lösung dieser Aufgaben erfolgt erfindungsgemäß mit den Merkmalen der unabhängigen Patentansprüche 1 und 15. Vorteilhafte Ausführungsformen sind Gegenstand der Unteransprüche.

Nach der Lehre der Erfindung werden diese Aufgaben gelöst durch Messen des Drucks in einem geschlossenen, zumindest teilweise mit Luft gefüllten Behälter mit einer ersten Druckmessung, Hineinfördern eines ersten Flüssigkeitsvolumens mit einem ersten Fördermittel in den Behälter, Messen des Drucks in dem Behälter mit einer zweiten Druckmessung nach dem Hineinfördern des ersten Flüssigkeitsvolumens in den Behälter, Herausfördern eines zweiten Flüssigkeitsvolumens mit einem zweiten Fördermittel aus dem Behälter, Messen des Drucks in dem Behälter mit einer dritten Druckmessung nach dem Herausfördern des zweiten Flüssigkeitsvolumens aus dem Behälter und Auswerten mindestens der Messwerte der ersten Druckmessung und der dritten Druckmessung als Maß für die Abweichung der Förderleistung des zweiten Fördermittels von der Förderleistung des ersten Fördermittels. Durch das Hineinfördern des ersten Flüssigkeitsvolumens wird das Luftvolumen in dem Behälter komprimiert und der Druck steigt von dem Messwert der ersten Druckmessung auf den Messwert der zweiten Druckmessung an. Durch das Herausfördern des zweiten Flüssigkeitsvolumens wird das komprimierte Luftvolumen wieder entspannt und der Druck in dem Behälter sinkt von dem Messwert der zweiten Druckmessung auf den Messwert der dritten Druckmessung. Diese Reihenfolge der Fördervorgänge, nämlich zuerst Fördern in den Behälter hinein und danach Fördern aus dem Behälter heraus, wird als erste Förderreihenfolge definiert.

Von der Lehre der Erfindung wird auch der Sonderfall erfasst, dass sich in dem Behälter keine Luft befindet, sondern die Elastizität des Behälters und ggf. der Leitungen ausreichend groß ausgelegt ist, um das geförderte Flüssigkeitsvolumen aufzunehmen. Dieser Sonderfall kann zur Anwendung kommen, wenn sehr kleine Fördervolumina mit hoher Genauigkeit gemessen werden sollen. Ausgehend von diesem Sonderfall kann durch das Vorsehen eines geeigneten Luftvolumens in dem Behälter die Compliance an die im Anwendungsfall vorliegenden Behältervolumina und Drücke angepasst werden.

Natürlich kann erfindungsgemäß auch in umgekehrter Reihenfolge verfahren werden, indem nach der Lehre der Erfindung die Aufgaben gelöst werden durch Messen des Drucks in einem geschlossenen, zumindest teilweise mit Luft und zumindest teilweise mit Flüssigkeit gefüllten Behälter mit einer ersten Druckmessung, Herausfördern eines ersten Flüssigkeitsvolumens mit einem ersten Fördermittel aus dem Behälter, Messen des Drucks in dem Behälter mit einer zweiten Druckmessung nach dem Herausfördern des ersten Flüssigkeitsvolumens aus dem Behälter, Hineinfördern eines zweiten Flüssigkeitsvolumens mit einem zweiten Fördermittel in den geschlossenen Behälter, Messen des Drucks in dem Behälter mit einer dritten Druckmessung nach dem Hineinfördern des zweiten Flüssigkeitsvolumens in den Behälter und Auswerten mindestens der Messwerte der ersten Druckmessung und der dritten Druckmessung als Maß für die Abweichung der Förderleistung des zweiten Fördermittels von der Förderleistung des ersten Fördermittels. Bei dieser Reihenfolge ist natürlich vor dem Herausfördern eines ersten Flüssigkeitsvolumens mit einem ersten Fördermittel aus dem Behälter ein ausreichendes Flüssigkeitsvolumen in dem Behälter erforderlich. Durch das Herausfördern des ersten Flüssigkeitsvolumens wird das Luftvolumen in dem Behälter entspannt und der Druck sinkt von dem Messwert der ersten Druckmessung auf den Messwert der zweiten Druckmessung. Durch das Hineinfördern des zweiten Flüssigkeitsvolumens wird das entspannte Luftvolumen wieder komprimiert und der Druck in dem Behälter steigt von dem Messwert der zweiten Druckmessung auf den Messwert der dritten Druckmessung an. Diese Reihenfolge der Fördervorgänge, nämlich zuerst Herausfördern aus dem Behälter und danach Hineinfördern in den Behälter, wird als zweite Förderreihenfolge definiert.

Darüber hinaus kann in manchen Ausführungsformen das Überprüfen der Förderleistung zusätzlich das Korrigieren der Förderleistung umfassen. Unter Korrigieren kann Beheben einer fehlerhaften Abweichung zu verstehen sein. Unter Korrigieren kann insbesondere das Erhöhen oder Senken der Förderleistung bis zum Erreichen eines Sollwerts zu verstehen sein. Das Korrigieren der Förderleistung kann durch einen Stelleingriff am Fördermittel oder an dem elektrischen Antrieb des Fördermittels erfolgen.

Als Kriterium zum Überprüfen der Förderleistung des ersten Fördermittels und/oder des zweiten Fördermittels kann erfindungsgemäß eine Abweichung der Förderleistung des ersten Fördermittels von der Förderleistung des zweiten Fördermittels oder lediglich ein Maß für eine Abweichung der Förderleistung des ersten Fördermittels von der Förderleistung des zweiten Fördermittels ermittelt werden. Ein Maß für eine Abweichung der Förderleistung des ersten Fördermittels von der Förderleistung des zweiten Fördermittels kann erfindungsgemäß beispielsweise ein gemessener Druck, eine berechnete Druckdifferenz aus gemessenen Drücken oder eine berechnete Volumendifferenz aus dem geförderten ersten und zweiten Flüssigkeitsvolumen sein.

In bestimmten Ausführungsformen kann ein Maß für eine Abweichung der Förderleistung durch Vergleich mindestens eines gemessenen Drucks mit einem unteren Grenzwert und/oder einem oberen Grenzwert erhalten werden. Bei Unterscheiten des unteren Grenzwerts oder Überschreiten des oberen Grenzwerts kann auf eine unzulässige Abweichung der Förderleistung mindestens des ersten

und/oder des zweiten Fördermittels geschlossen werden. Beispielsweise können mindestens die Messwerte der ersten Druckmessung und der dritten Druckmessung verglichen werden.

Das Auswerten mindestens der Messwerte der ersten Druckmessung und der dritten Druckmessung kann anderen Ausführungsformen das Vergleichen des Messwerts der dritten Druckmessung mit einem oberen Grenzwert und/oder einem unteren Grenzwert umfassen.

Der obere Grenzwert und der unteren Grenzwert können beispielsweise als zulässige Abweichungen nach oben bzw. nach unten in Bezug auf den Messwert der ersten Druckmessung oder auf einen vorgegebenen Drucksollwert definiert werden.

Alleine aufgrund des Auswertens mindestens der Messwerte der ersten Druckmessung und der dritten Druckmessung kann zunächst nicht bestimmt werden, ob das erste oder das zweite Fördermittel oder sogar beide Fördermittel eine fehlerhafte Förderleistung aufweisen und welche Beträge die Abweichungen aufweisen. Jedoch kann festgestellt werden, ob die Förderleistung des zweiten Fördermittels in unzulässigem Maß von der Förderleistung des ersten Fördermittels oder die Förderleistung des ersten Fördermittels in unzulässigem Maß von der Förderleistung des zweiten Fördermittels abweicht.

In einem ersten Fehlerfall kann bei Anwendung der ersten Förderreihenfolge festgestellt werden, dass die Förderleistung des zweiten Fördermittels die Förderleistung des ersten Fördermittels unzulässig überschreitet, wenn die dritte Druckmessung den unteren Druckgrenzwert unterschreitet. In einem zweiten Fehlerfall kann festgestellt werden, dass die Förderleistung des zweiten Fördermittels die Förderleistung des ersten Fördermittels unzulässig unterschreitet, wenn die dritte Druckmessung den oberen Druckgrenzwert überschreitet.

Bei Anwendung der zweiten Förderreihenfolge kann in einem ersten Fehlerfall festgestellt werden, dass die Förderleistung des zweiten Fördermittels die Förderleistung des ersten Fördermittels unzulässig überschreitet, wenn die dritte Druckmessung den oberen Druckgrenzwert überschreitet. In einem zweiten Fehlerfall kann festgestellt werden, dass die Förderleistung des zweiten Fördermittels die Förderleistung des ersten Fördermittels unzulässig unterschreitet, wenn die dritte Druckmessung den unteren Druckgrenzwert unterschreitet.

Im einfachsten Fall sind das erste Fördermittel und das zweite Fördermittel baugleich und weisen deshalb die gleiche Nennförderleistung auf. In anderen Fällen können das erste Fördermittel und das zweite Fördermittel unterschiedliche Nennförderleitungen aufweisen. Die Festlegung des unteren Grenzwerts und des oberen Grenzwerts definiert einen Toleranzbereich des Drucks in dem Behälter allein zur Berücksichtigung der zulässigen Abweichungen der Förderleistung der beiden Fördermittel von der Nennförderleistung.

Die Abweichungen der Förderleistung der beiden Fördermittel können konstruktiv bedingt sein. Bekannte Unterschiede der beiden Fördermittel können beispielsweise durch unterschiedliche Nennförderleistungen gegeben sein, wenn das erste Fördermittel und das zweite Fördermittel nicht baugleich sind. Die Festlegung des unteren Grenzwerts und des oberen Grenzwerts definiert einen Toleranzbereich der zulässigen Abweichung. Dieser Toleranzbereich kann bei nicht baugleichen Fördermitteln zum einen zulässige Abweichungen der Förderleistungen der beiden Fördermittel von deren Nennförderleistungen berücksichtigen. Außerdem kann der Toleranzbereich durch unterschiedliche Auslegungsparameter der beiden Fördermittel bedingte Unterschiede berücksichtigen. Ein Beispiel für unterschiedliche Auslegungsparameter der beiden Fördermittel sind unterschiedliche Hubvolumina bei zwei Membranpumpen.

Als Maß für eine Abweichung der Förderleistung kann in anderen Ausführungsformen eine Druckdifferenz ermittelt werden, beispielsweise eine berechnete Druckdifferenz aus dem Messwert der ersten Druckmessung und dem Messwert der dritten Druckmessung. Die Druckdifferenz kann mit einem unteren Grenzwert und/oder einem oberen Grenzwert verglichen werden. Bei Unterscheiten des unteren Grenzwerts oder Überschreiten des oberen Grenzwerts kann auf eine unzulässige Abweichung der Förderleistung mindestens des ersten und/oder des zweiten Fördermittels geschlossen werden.

In manchen Ausführungsformen kann anstelle des Absolutdrucks der Relativdruck in dem Behälter gemessen werden. Dies gilt beispielsweise für Ausführungsformen die eine Auswertung der Messwerte der ersten Druckmessung und der dritten Druckmessung und den Vergleich des Messwerts der dritten Druckmessung mit einem unteren Grenzwert und/oder einem oberen Grenzwert vorsehen. Dies gilt weiterhin beispielsweise für andere Ausführungsformen, die das Vergleichen der Druckdifferenz mit einem oberen Grenzwert und/oder einem unteren Grenzwert umfassen.

Ein Maß für eine Abweichung der Förderleistung kann in wiederum anderen Ausführungsformen eine Volumendifferenz sein. Die Volumendifferenz kann mit einem unteren Grenzwert und/oder einem oberen Grenzwerts verglichen werden. Bei Unterschreiten des unteren Grenzwerts oder Überschreiten des oberen Grenzwerts kann auf eine unzulässige Abweichung der Förderleistung des ersten und/oder des zweiten Fördermittels geschlossen werden. Anstelle des Vergleichs des dritten Druckmesswerts mit einem unteren und einem oberen Druckgrenzwert kann bei solchen Ausführungsformen zunächst auf der Basis des Gesetzes von Boyle-Mariotte die Berechnung einer Volumendifferenz aus dem Druckmesswerten erfolgen. Die Fördervolumendifferenz kann positiv oder negativ sein. Für solche Ausführungsformen ist die Messung des Absolutdrucks in dem Behälter erforderlich. Als Mittel zum Messen des Drucks kommt ein Absolutdruckmesser zum Einsatz.

Für die Zustandsänderung in dem Behälter aufgrund des Förderns des ersten Flüssigkeitsvolumens gilt:

$$P_0 \cdot V_{vessel,air,0} = P_1 \cdot (V_{vessel,air,0} - V_{pump,1}) \qquad (1)$$

Für die Zustandsänderung in dem Behälter aufgrund des Förderns des zweiten Flüssigkeitsvolumens gilt:

$$P_0 \cdot V_{vessel,air,0} = P_2 \cdot (V_{vessel,air,0} - V_{pump,1} + V_{pump,2}) \qquad (2)$$

In der Gleichungen (1) und (2) bedeutet $P_0$ den Absolutdruck in dem Behälter vor dem Fördern des ersten Flüssigkeitsvolumens, $V_{vessel,air,0}$ das initiale Luftvolumen in dem Behälter, $V_{pump,1}$ das erste Flüssigkeitsvolumen nach dem Fördern mit einem ersten Fördermittel, $V_{pump,2}$ das zweite Flüssigkeitsvolumen nach dem Fördern mit einem zweiten Fördermittel, $P_1$ den Absolutdruck in dem Behälter in der Mischkammer nach dem Fördern des ersten Flüssigkeitsvolumens und $P_2$ den Absolutdruck in der Mischkammer nach dem Fördern des zweiten Flüssigkeitsvolumens.

Aufgrund einer berechneten Druckdifferenz nach Gleichung (3) kann auf einen Fördervolumenfehler nach Gleichung (4) geschlossen werden.

$$\Delta P = P_2 - P_0 \quad (3)$$

$$\Delta V = V_{pump,1} - V_{pump,2} \qquad (4)$$

Mit Hilfe der Definition der Compliance nach Gleichung (5) kann der Fördervolumenfehler nach Gleichung (6) berechnet werden.

$$C = \frac{\Delta V}{\Delta P} = \frac{V_{vessel,air,0}}{P_0} \qquad (5)$$

$$\Delta V = C \cdot (P_2 - P_0) \quad (6)$$

Die Compliance kann als Systemparameter bekannt sein. Das initiale Luftvolumen in dem Behälter kann durch eine Füllstandsmessung ermittelt werden. Die Drücke können durch einen Absolutdrucksensor gemessen werden.

Der Fördervolumenfehler kann mit einem oberen Volumenfehlergrenzwert und einem unteren Volumenfehlergrenzwert verglichen werden. Es ist alternativ auch möglich, lediglich den Betrag des Fördervolumenfehlers mit nur einem Volumenfehlergrenzwert zu vergleichen. Die Volumenfehlergrenzwerte können als Abweichungen nach oben und/oder nach unten bezogen auf einen Sollwert mindestens eines Fördervolumens vorgegeben werden. Die Abweichungen noch oben und/oder nach unten können auf den Sollwert des Fördervolumens mindestens des ersten oder des zweiten Fördermittels bezogen werden.

In einem ersten Fehlerfall kann bei Anwendung der ersten Förderreihenfolge und unter Vorgabe eines oberen Grenzwerts und eines unteren Grenzwerts jeweils definiert als positive und als negative Abweichung vom Sollwert des Fördervolumens des ersten Fördermittels festgestellt werden, dass das Fördervolumen des zweiten Fördermittels das Fördervolumen des ersten Fördermittels unzulässig überschreitet, wenn der Volumenfehler nach Gleichung (6) den unteren Volumenfehlergrenzwert unterschreitet. In einem zweiten Fehlerfall kann festgestellt werden, dass das Fördervolumen des zweiten Fördermittels das Fördervolumen des ersten Fördermittel unzulässig unterschreitet, wenn die Fördervolumendifferenz den oberen Volumenfehlergrenzwert überschreitet. Im ersten und im zweiten Fehlerfall kann festgestellt werden, dass mindestens das erste oder das zweiter Fördermittel oder beide Fördermittel eine fehlerhafte Förderleistung aufweisen. Dabei kann allerdings zunächst nicht festgestellt werden, welches der Fördermittel die fehlerhafte Förderleistung aufweist, wobei dies zur Fehlererkennung zunächst ausreichend ist.

Analog dazu kann bei Anwendung der zweiten Förderreihenfolge in einem ersten Fehlerfall festgestellt werden, dass das Fördervolumen des zweiten Fördermittels das Fördervolumen des ersten Fördermittels unzulässig überschreitet, wenn der Volumenfehler nach Gleichung (6) den oberen Volumenfehlergrenzwert überschreitet. In einem zweiten Fehlerfall kann festgestellt werden, dass das Fördervolumen des zweiten Fördermittels das Fördervolumen des ersten Fördermittels unzulässig unterschreitet, wenn die Fördervolumendifferenz den unteren Volumenfehlergrenzwert unterschreitet.

Das Fördern eines ersten Flüssigkeitsvolumens und/oder eines zweiten Fördervolumens kann diskontinuierlich oder kontinuierlich erfolgen. Unter diskontinuierlichem Fördern kann ein Fördern mittels wiederholter Pumpenhübe verstanden werden, wobei die Förderung während eines einzelnen Pumpenhubs wiederum kontinuierlich erfolgt.

Beispiele für diskontinuierlich fördernde Pumpen sind Membranpumpen und Kolbenpumpen. Die Förderleistung wird bei diesen Pumpen, die ein konstantes Hubvolumen aufweisen können, durch die Hubanzahl pro Zeiteinheit eingestellt. Ein bestimmtes Fördervolumen wird durch eine bestimmte Anzahl von Förderhüben gefördert.

Ein weiteres Beispiel für eine diskontinuierlich fördernde Pumpe ist eine Pumpe mit einem Schrittmotor, der schrittweise, also diskontinuierlich arbeitet. Die Förderleistung kann bei einer solchen Pumpe beispielsweise mittels einer Steuer- und Recheneinheit durch Vorgeben eines Schrittwinkels pro Förderschritt und einer Schrittanzahl pro Zeiteinheit an den Schrittmotor eingestellt werden. Ein bestimmtes Fördervolumen kann bei einer solchen Pumpe beispielsweise mittels einer Steuer- und Recheneinheit durch Vorgeben eines Schrittwinkels pro Förderschritt und einer Schrittanzahl an den Schrittmotor eingestellt werden. Allerdings ist es für eine genaue Förderung erforderlich, dass die Pumpe okkludierend arbeitet, so dass es nicht zu Rückströmungen kommen kann.

Eine kontinuierliche Förderung eines bestimmten Fördemolumens mit einer Zahnradpumpe kann beispielsweise durch Vorgeben einer Drehzahl und einer Förderzeit an einen kontinuierlich drehenden Antriebsmotor, beispielsweise einen bürstenlosen Gleichstrommotor, mittels einer Steuer- und Recheneinheit eingestellt werden.

Im Folgenden wird ein Ausführungsbeispiel der Erfindung unter Bezugnahme auf die Figuren näher erläutert. Anhand des in den Figuren dargestellten Ausführungsbeispiels werden weitere Einzelheiten und Vorteile der Erfindung näher beschrieben. Die erfindungsgemäße Vorrichtung wird am Beispiel einer als Hämodialysevorrichtung ausgestalteten Blutbehandlungsvorrichtung beschrieben.

Es zeigen:

Figur 1 ein Flussbild des Dialyatsystems einer als Hämodialysevorrichtung ausgestalteten Blutbehandlungsvorrichtung mit einer Mischkammer;

Figur 2 eine grafische Darstellung der Druckänderungen in der Mischkammer des Dialysierflüssigkeitssystems aus Figur 1 bei der Durchführung des Verfahrens mit zwei Fördermitteln mit ordnungsgemäßer Förderleistung;

Figur 3 eine grafische Darstellung der Druckänderungen in der Mischkammer des Dialysierflüssigkeitssystems aus Figur 1 bei der Durchführung des Verfahrens mit zwei Fördermitteln mit fehlerhafter Förderleistung mindestens des ersten oder des zweiten Fördermittels und Überschreiten des oberen Grenzwerts für den Relativdruck;

Figur 4 eine grafische Darstellung der Druckverhältnisse des Relativdrucks in der Mischkammer des Dialysierflüssigkeitssystems aus Figur 1 bei der Durchführung des Verfahrens mit zwei Fördermitteln mit fehlerhafter Förderleistung mindestens des ersten oder des zweiten Fördermittels und Unterschreiten des unteren Grenzwerts für den Relativdruck.

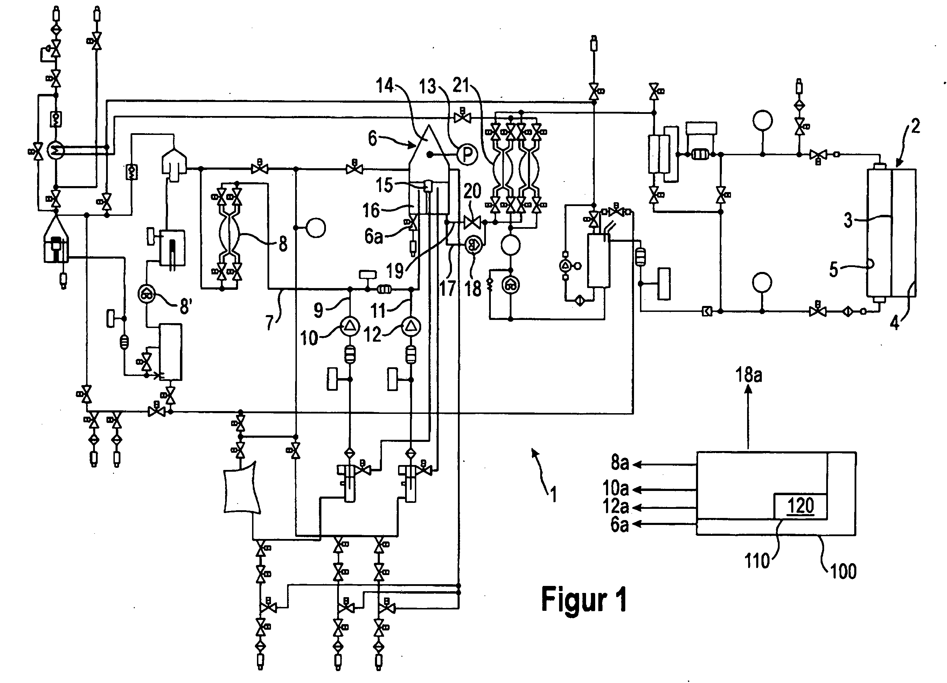

Figur 1 zeigt in vereinfachter schematischer Darstellung die wesentlichen Komponenten des Dialysierflüssigkeitssystems 1 einer als Hämodialysevorrichtung ausgestalteten Blutbehandlungsvorrichtung. Bei dem vorliegenden Ausführungsbeispiel ist die BlutbehandlungsVorrichtung eine Hämodialysevorrichtung, die einen Dialysator 2 aufweist, der schematisch durch eine semipermeable Membran 3 in eine Blutkammer 4 und einer Dialysierflüssigkeitskammer 5 getrennt ist. Die Blutkammer ist Teil des extrakorporalen Blutkreislaufs (nicht gezeigt) und die Dialysierflüssigkeitskammer 5 ist Teil des Dialysierflüssigkeitssystems 1. Die zentrale Steuer- und Recheneinheit 100 betreibt und überwacht die Blutbehandlungsvorrichtung und das Dialysierflüssigkeitssystem. Die Steuer- und Recheneinheit 110 der erfindungsgemäßen Vorrichtung ist im Ausführungsbeispiel Teil der zentralen Steuer- und Recheneinheit 100 der Blutbehandlungsvorrichtung. Die Steuer- und Recheneinheit 110 kann aber auch getrennt von der zentralen Steuereinheit 100 sein und mit dieser über Datenleitungen verbunden sein.

Das Dialysierflüssigkeitssystem verfügt über eine Mischkammer 6 zum Anmischen frischer Dialysierflüssigkeit aus Reinwasser und Flüssigkonzentraten. Das Dialysierflüssigkeitssystem verfügt über eine Leitung 7 zum Führen von Reinwasser, in die eine passive Membrankammer 8 geschaltet ist, die erst im Zusammenwirken mit einer stromaufwärts angeordneten Zahnradpumpe $8^I$ (Entgasungspumpe) ein Fördermittel mit Dosierfunktion bildet, dass nachfolgend als Fördermittel 8 bezeichnet wird. Die Leitung 7 mündet in die Mischkammer 6. Bei dem Fördermittel 8 handelt es sich um ein Fördermittel für Reinwasser.

Eine weitere Leitung 9 mündet stromabwärts des Fördermittels 8 in die Leitung 7. In die Leitung 9 ist ein Fördermittel 10 geschaltet. Bei dem Fördermittel 10 handelt es sich in dem Ausführungsbeispiel um eine Dosierpumpe für Natriumbicarbonat-Konzentrat. Die Dosierpumpe 10 ist als Membranpumpe ausgeführt.

Eine wiederum weitere Leitung 11 mündet an anderer Stelle ebenfalls stromabwärts des Fördermittels 8 in die Leitung 7. In die Leitung 11 ist ein Fördermittel 12 geschaltet. Bei dem Fördermittel 12 handelt es sich in dem Ausführungsbeispiel um eine Dosierpumpe für Säure-Konzentrat. Die Dosierpumpe 12 ist als Membranpumpe ausgeführt.

Grundsätzlich kann im Ausführungsbeispiel für die Durchführung des Verfahrens das Fördermittel 8, das Fördermittel 10 oder das Fördermittel 12 zum Fördern einer Flüssigkeit in den Mischbehälter benutzt werden. Im Ausführungsbeispiel wird die Durchführung des Verfahrens mit dem Fördermittel 10 als erstem Fördermittel erläutert.

In anderen Ausführungsformen können erfindungsgemäß beliebige andere stromaufwärts der Mischkammer 6 abgeordnete Pumpen als erstes Fördermittel ausgewählt werden. Die Auswahl des ersten Fördermittels kann automatisch durch die Steuer- und Recheneinheit erfolgen.

Eine Leitung 17 führt stromabwärts von der Mischkammer 6 zur Dialysierflüssigkeitskammer 5 des Dialysators 2. In die Leitung 17 ist ein Fördermittel 18 geschaltet. Bei dem Fördermittel handelt es sich in dem Ausführungsbeispiel um eine Zahnradpumpe 18, die Teil einer Bilanziervorrichtung 21 ist. In Parallelschaltung zur Leitung 17 befindet sich eine Bypass-Leitung 19 mit einem Bypass-Ventil 20. Das Bypass-Ventil 20 ist während des Betriebs der Zahnradpumpe 18 geschlossen.

Im Ausführungsbeispiel wird die erfindungsgemäße Vorrichtung in Verbindung mit der Zahnradpumpe 18 als zweitem Fördermittel beschrieben.

In anderen Ausführungsformen können erfindungsgemäß beliebige andere stromabwärts der Mischkammer 6 abgeordnete Pumpen in als zweites Fördermittel ausgewählt werden. Die Auswahl des zweiten Fördermittels kann automatisch durch die Steuer- und Recheneinheit erfolgen.

Die Steuer- und Recheneinheit 110 kann Mittel zum Auswählen eines der Fördermittel (8, 10, 12) zum Fördern eines ersten Fördervolumens in die Mischkammer 6 aufweisen. Die Auswahl kann in der Steuer- und Recheneinheit 110 fest eingestellt sein oder durch den Anwender durch Benutzereingriff, beispielsweise über den Touchscreen (in Figur 1 nicht gezeigt) der Blutbehandlungsvorrichtung, eingestellt werden.

Die Steuer- und Recheneinheit 110 kann außerdem Mittel zum Auswählen eines der stromabwärts der Mischkammer angeordneten Fördermittel, beispielsweise die Zahnradpumpe 18, oder andere stromabwärts der Mischkammer angeordnete Pumpen (in Figur 1 nicht gezeigt) zum Herausfördern eines zweiten Fördervolumens aus der Mischkammer 6 aufweisen. Die Auswahl kann wiederum in der Steuer- und Recheneinheit 110 fest eingestellt sein oder durch den Anwender durch Benutzereingriff, beispielsweise über den Touchscreen der Blutbehandlungsvorrichtung (in Figur 1 nicht gezeigt), eingestellt werden.

In dem Ausführungsbeispiel wird die Membranpumpe 10 von der Steuer- und Recheneinheit 110 als erstes Fördermittel ausgewählt. Als zweites Fördermittel wird die Zahnradpumpe 18 ausgewählt. Ein Förderhub der Membranpumpe 10 entspricht dem Hubvolumen, das bei einem vollständigen Pumpenhub gefördert wird. Der Förderhub könnte alternativ aber auch einen Teil eines vollständigen Pumpenhubs umfassen, beispielsweise wenn der Pumpenantrieb ein Schrittmotor ist.

Die Streuer- und Recheneinheit 110 weist Mittel zum Veranlassen der Förderung eines vorgegebenen Fördervolumens des ersten Fördermittels auf, beispielsweise durch Vorgabe einer Anzahl von Förderhüben der Membranpumpe 10. Die Förderhübe werden durch Steuereingriffe 10a veranlasst. Außerdem weist die Steuer- und Recheneinheit 110 Mittel zum Veranlassen der Förderung eines vorgegebenen Fördervolumens des zweiten Fördermittels, beispielsweise durch Vorgabe eines Schrittwinkels pro Förderschritt und einer Schrittanzahl mittels der Steuereingriffe 18a an den Schrittmotor der Zahnradpumpe 18 auf.

Die erfindungsgemäße Vorrichtung weist einen Drucksensor 13 auf, der den relativen Druck in der Mischkammer 6 misst. Die Messwerte des Drucksensors 13 werden an die Steuer- und Recheneinheit 110 übertragen und liegen dort in dem Datenspeicher 120 zur Auswertung vor.

Des Weiteren weist die Steuer- und Recheneinheit 110 einen Datenspeicher 120 auf.

Die Berechnungen der gesuchten Druckdifferenzen erfolgen mit Hilfe eines Computerprogramms mit Programmcode zur Veranlassung der maschinellen Schritte des Verfahrens und zur Auswertung des Messergebnisses. Die Berechnungsgleichungen sind in dem Programmcode implementiert. Der Programmcode ist in der Steuer- und Recheneinheit 110 gespeichert.

Die Steuer- und Recheneinheit 110 verfügt über einen Datenspeicher 120.

Das Computerprögramm mit Programmcode zur Veranlassung der maschinellen Schritte des Verfahrens und zur Auswertung des Messergebnisses startet die Berechnungen, sobald die Förderung des vorgegebenen ersten und zweiten Fördervolumens abgeschlossen ist.

Das gesamte Innenvolumen der Mischkammer 6 ist bekannt und beträgt in dem Ausführungsbeispiel 350 ml. Das initiale Flüssigkeitsvolumen 16 in der Mischkammer 6 wird aus dem Messwert der Füllstandsmessvorrichtung 15 berechnet. Das initiale Luftvolumen 14 in der Mischkammer wird von der Steuer- und Recheneinheit 110 als Differenz des gesamten Innenvolumens und des Flüssigkeitsvolumens berechnet und beträgt in dem Ausführungsbeispiel 242 ml. Die Temperatur in der Mischkammer beträgt 37 °C und wird während der Durchführung des Verfahrens als konstant angenommen.

Die Anzahl der durchzuführenden Förderhübe des ersten Fördermittels ist in dem Ausführungsbeispiel mit m = 50 in der zentrale Steuerung der Blutbehandlungsvorrichtung vorgegeben. Das Hubvolumen der Membranpumpe 10 beträgt in dem Ausführungsbeispiel 1 ml und entspricht dem Fördervolumen eines einzelnen vollständigen Förderhubs der Membranpumpe 10. Die Steuer- und Recheneinheit 110 startet und stoppt die Förderhübe der Membranpumpe durch Steuereingriffe 10a. Es wird mit jedem Förderhub 1 ml Flüssigkeit in die Mischkammer 6 gepumpt. Eine Rückströmung der Flüssigkeit durch die Membranpumpe 10 findet nicht statt. Der Druck in der Mischkammer erhöht sich mit jedem Förderhub entsprechend dem Gesetz von Boyle-Mariotte, weil das Flüssigkeitsvolumen mit jedem Förderhub um den Betrag eines Förderhubs zunimmt und im Gegenzug das Luftvolumen mit jedem Förderhub in gleichem Maße um den Betrag des Förderhubs abnimmt. Die Luft wird deshalb mit jedem Förderhub um denselben Betrag komprimiert. Dieser Zusammenhang wird durch die Gleichung (7) beschrieben. Die thermodynamische Grundlage der Gleichung (7) ist das Gesetz von Boyle-Mariotte, angewendet auf die durch die Förderhübe verursachten Zustandsänderungen des Luftvolumens in dem Mischbehälter.

$$P_0 \cdot V_{vessel,air,0} = P_1 \cdot (V_{vessel,air,0} - m \cdot V_{pump,1}) \quad (7)$$

Die Anzahl der durchzuführenden Förderschritte des zweiten Fördermittels ist in dem Ausführungsbeispiel ebenfalls mit n = 50 in der zentrale Steuerung der Blutbehandlungsvorrichtung vorgegeben. Das Fördervolumen pro Förderschritt der Zahnradpumpe 18 beträgt in dem Ausführungsbeispiel ebenfalls 1 ml und entspricht somit dem Fördervolumen eines einzelnen Förderhubs der Membranpumpe 10. Die Steuer- und Recheneinheit 110 startet und stoppt durch Ansteuern des Schrittmotors der Zahnradpumpe 18 mit Steuereingriffen 18a die Förderschritte. Es wird mit jedem Förderschritt der Zahnradpumpe 18 1 ml Flüssigkeit aus der Mischkammer 6 gepumpt. Eine Rückströmung der Flüssigkeit durch die Zahnradpumpe 18 findet nicht statt. Der Druck in der Mischkammer verringert sich mit jedem Förderschritt der Zahnradpumpe 18 entsprechend dem Gesetz von Boyle-Mariotte, weil das Flüssigkeitsvolumen in der Mischkammer mit jedem Förderschritt abnimmt und im Gegenzug das Luftvolumen mit jedem Förderschritt in gleichem Maße entspannt wird. Die Luft wird deshalb mit jedem Förderschritt um denselben Betrag entspannt. Dieser Zusammenhang wird durch die Gleichung (8) beschrieben.

$$P_0 \cdot V_{vessel,air,0} = P_2 \cdot (V_{vessel,air,0} - m \cdot V_{pump,1} + n \cdot V_{pump,2}) \quad (8)$$

In der Gleichungen (7) und (8) bedeutet $P_0$ den Absolutdruck in dem Behälter vor dem Fördern des ersten Flüssigkeitsvolumens, $V_{vessel,air,0}$ das initiale Luftvolumen in dem Behälter, $V_{pump,1}$ das Hubvolumen der ersten Membranpumpe 10, $V_{pump,2}$ das Fördervolumen pro Förderschritt der Zahnradpumpe 18, $P_1$ den Absolutdruck in dem Behälter in der Mischkammer nach dem Fördern des ersten Flüssigkeitsvolumens, $P_2$ den Absolutdruck in der Mischkammer nach dem Fördern des zweiten Flüssigkeitsvolumens, m die Anzahl der Pumpenhübe der ersten Membranpumpe 10 und n die Schrittanzahl der Zahnradpumpe 18, wobei m gleich n sein kann.

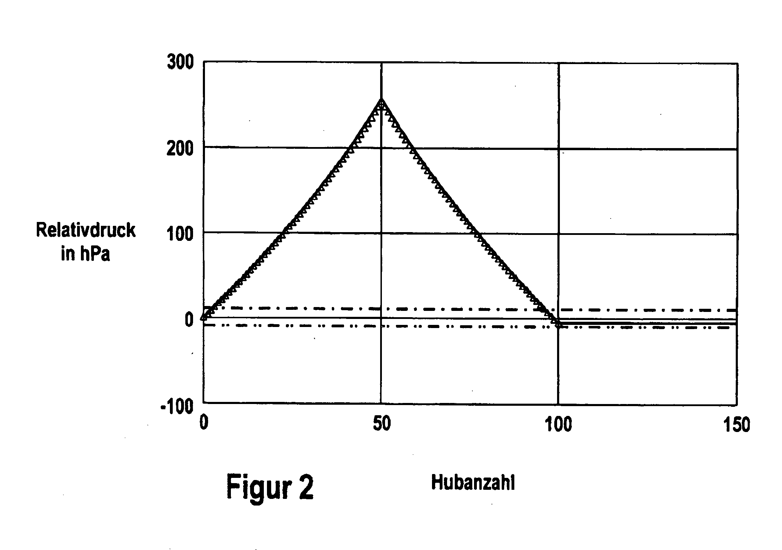

Eine erste erfindungsgemäße Überprüfung der Förderleistungen mit ordnungsgemäßem Hubvolumen beider Fördermittel 10 und 18 ergibt den in der Figur 2 gezeigten Verlauf der Druckänderungen in der Mischkammer 6 des Dialysierflüssigkeitssystems aus Figur 1. Aus der Figur 2 ist ersichtlich, dass das Fördervolumen der Zahnradpumpe 18 geringfügig größer ist als das Fördervolumen der Membranpumpe 10, weil der Druck nach dem zweiten Fördervorgang kleiner ist als der Druck vor dem ersten Fördervorgang. Der Druck nach dem zweiten Fördervorgang liegt jedoch über dem unteren Druckgrenzwert und unter dem oberen Druckgrenzwert. Es wird daraus geschlossen, dass der Volumenfehler innerhalb des Toleranzbereichs liegt. Die Förderleistungen der Pumpen 10 und 18 ergeben keinen Volumenfehler. Da die Wahrscheinlichkeit des gleichzeitigen Versagens beider Pumpen 10 und 18 hinreichend klein ist, wird geschlossen, dass die Förderleistungen beider Membranpumpen 10 und 18 ordnungsgemäß sind.

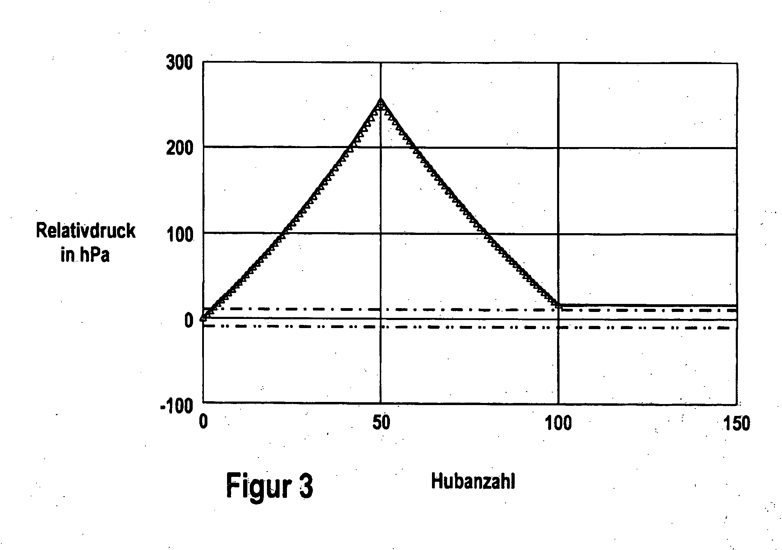

Eine zweite erfindungsgemäße Überprüfung der Förderleistungen mit fehlerhaftem Hubvolumen einer der beiden Pumpen 10 und 18 ergibt den in der Figur 3 gezeigten Verlauf der Druckänderungen in der Mischkammer des Dialysierflüssigkeitssystems aus Figur 1. Aus der Figur 3 ist ersichtlich, dass das Fördervolumen der Zahnradpumpe 18 kleiner ist als das Fördervolumen der Membranpumpe 10, weil der Druck nach dem zweiten Fördervorgang größer ist als der Druck vor dem ersten Fördervorgang. Der Druck nach dem zweiten Fördervorgang liegt über dem oberen Druckgrenzwert. Es wird daraus geschlossen, dass der Volumenfehler außerhalb des Toleranzbereichs liegt und mindestens eine der Pumpen 10, 18 fehlerhaft ist.

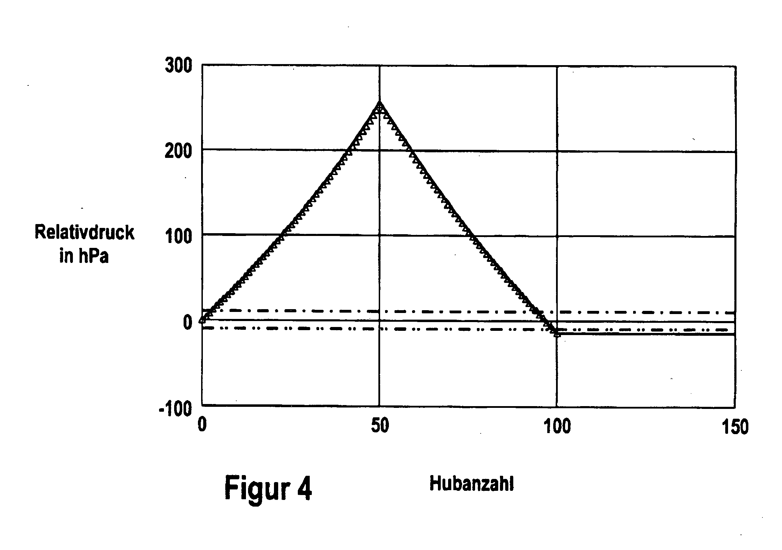

Eine dritte erfindungsgemäße Überprüfung der Förderleistungen mit fehlerhaftem Fördervolumen einer der beiden Pumpen 10 und 18 ergibt den in der Figur 4 gezeigten Verlauf der Druckänderungen in der Mischkammer des Dialysierflüssigkeitssystems aus Figur 1. Aus der Figur 4 ist ersichtlich, dass das Fördervolumen der Zahnradpumpe 18 größer ist als das Fördervolumen der Membranpumpe 10, weil der Druck nach dem zweiten Fördervorgang kleiner ist als der Druck vor dem ersten Fördervorgang. Der Druck nach dem zweiten Fördervorgang liegt unter dem unteren Druckgrenzwert. Es wird daraus geschlossen, dass der Volumenfehler außerhalb des Toleranzbereichs liegt und mindestens eine der Pumpen 10, 18 fehlerhaft ist.

Bei der Membranpumpe 10 handelt es sich um ein bekanntermaßen sehr zuverlässiges Fördermittel, dessen Förderleistung sich erfahrungsgemäß im Laufe der Betriebszeit nur sehr geringfügig ändert. Bei der Zahnradpumpe 18 kann sich die Förderleistung erfahrungsgemäß aufgrund des höheren Verschleisses der mechanischen Bauteile in relevantem Maße ändern. Die Förderleistung der Membranpumpe kann daher als Referenz herangezogen werden für die Überprüfung der Förderleistung der Zahnradpumpe 18. Anders ausgedrückt wird im vorliegenden Ausführungsbeispiel ein ermittelter Volumenfehler des ersten und/oder des zweiten Fördermittels ausschließlich der Zahnradpumpe 18 zugeordnet, weil die Wahrscheinlichkeit eines Fehlers der Zahnradpumpe 18 erfahrungsgemäß wesentlich höher ist als die Wahrscheinlichkeit eines Fehlers der Membranpumpe 10.

In einem weiteren Schritt kann nach Ermittlung eines unzulässigen Volumenfehlers von der Steuer- und Recheneinheit 110 ein Stelleingriff 18a am Pumpenantrieb der Zahnradpumpe 18 vorgenommen werden, beispielsweise durch eine Korrektur des Schrittwinkels, so dass der Volumenfehler automatisch korrigiert wird.

Es kann auch eine iterative Wiederholung der erfindungsgemäßen Überprüfung der Förderleistungen mit anschließender Korrektur erfolgen, bis der Volumenfehler korrigiert ist.

In analoger Weise kann ein Kreuzvergleich der Pumpen 8, 10, 12 mit der Pumpe 18 oder in anderen Ausführungsformen auch anderer Pumpen (in Figur 1 nicht gezeigt) stromaufwärts und stromabwärts der Mischkammer 6 durchgeführt werden. Dabei wird das Verfahren als Plausibilitätskontrolle mit mehreren Pumpenpaarungen durchgeführt. In dem Kreuzvergleich können beispielsweise in drei Kombinationen die Fördervolumina der Pumpen 8 und 18, 10 und 18 sowie 12 und 18 miteinander verglichen werden.

Wenn beispielsweise im vorliegenden Ausführungsbeispiel in dem Kreuzvergleich der Pumpen 10, 12 mit der Zahnradpumpe 18 in beiden Kombinationen jeweils ein Volumenfehler festgestellt wird, so wird der Volumenfehler der Zahnradpumpe 18 zugeordnet und es wird der Volumenfehler durch Stelleingriffe 18a am Pumpenantrieb der Zahnradpumpe 18 korrigiert.

Wenn jedoch in dem Kreuzvergleich der Pumpen 10, 12 mit der Zahnradpumpe 18 nur bei der einen Kombination ein Volumenfehler festgestellt wird und bei der anderen nicht, so wird eine Fehlermeldung ausgegeben und es wird kein weiterer Kreuzvergleich von Fördermitteln durchgeführt, weil eine Zuordnung des Volumenfehlers zur Zahnradpumpe 18 nicht plausibel ist. Es erfolgen demzufolge keine Stelleingriffe. Die zentrale Steuer- und Recheneinheit erhält in diesem Fehlerfall eine Fehlermeldung von der Steuer- und Recheneinheit 110, dass eine extrakorporale Blutbehandlung mit fehlerhaften Pumpen nicht durchgeführt oder nicht fortgeführt werden darf. In einem solchen Fall wäre eine herkömmliche Überprüfung der Pumpenleistung durch einen Servicetechniker erforderlich, weil mindestens eine der beiden Membranpumpen 10, 12 fehlerhaft ist. Ein Hinweis zur Anforderung eines Servicetechnikers kann auf dem Bildschirm der Blutbehandlungsvorrichtung angezeigt werden.

In allen Ausführungsformen kann von der Steuer- und Recheneinheit das Starten einer extrakorporalen Blutbehandlung unterbunden werden, wenn eine unzulässige Abweichung der Förderleistung eines oder mehrerer Fördermittel festgestellt wird. Dadurch kann die Sicherheit der extrakorporalen Blutbehandlung erhöht werden.

Erfindungsgemäß gelingt die Lösung der Aufgabenstellungen der vorliegenden Erfindung mit dem gezeigten Ausführungsbeispiel. Die vorliegende Erfindung ist jedoch nicht auf das Ausführungsbeispiel beschränkt.

| Bezugszeichen | Bezeichnung |
|---|---|
| | Bezugszeichenliste |
| 1 | Dialysierflüssigkeitssystem |

(fortgesetzt)

| Bezugzeichen | Bezeichnung |
|---|---|
| 2 | Dialysator |
| 3 | semipermeable Membran |
| 4 | Blutkammer |
| 5 | Dialysierflüssigkeitskammer |
| 6 | Mischkammer |
| 7 | erste Leitung |
| 8 | Membrankammer |
| $8^I$ | Zahnradpumpe |
| 8a | Stelleingriff |
| 9 | zweite Leitung |
| 10 | zweites Fördermittel, Dosierpumpe / Zahnradpumpe |
| 10a | Stelleingriff |
| 11 | dritte Leitung |
| 12 | drittes Fördermittel, Dosierpumpe / Zahnradpumpe |
| 12a | Stelleingriff |
| 13 | Drucksensor |
| 14 | initiales Luftvolumen |
| 15 | Füllstandsmessvorrichtung |
| 16 | Initiales Flüssigkeitsvolumen |
| 17 | Leitung |
| 18 | Zahnradpumpe |
| 18a | Stelleingriff |
| 19 | Bypass-Leitung |
| 20 | Bypass-Ventil |
| 21 | Bilanziervorrichtung |
| 100 | zentrale Steuer- und Recheneinheit |
| 110 | Steuer- und Recheneinheit |
| 120 | Daten speicher |

**Patentansprüche**

1. Vorrichtung zum Überprüfen der Förderleistung mindestens eines ersten (8, 10, 12) und/oder eines zweiten Fördermittels (18) einer Vorrichtung zur extrakorporalen Blutbehandlung mit
   einem geschlossenen, zumindest teilweise mit Luft gefüllten Behälter (6)
   **oder**
   einem geschlossenen, zumindest teilweise mit Luft und zumindest teilweise mit Flüssigkeit gefüllten Behälter (6),
   mindestens einem Mittel zum Messen des Drucks (13) in dem Behälter (6),
   einem mit dem Behälter (6) durch eine erste Flüssigkeitsleitung (7) in fluidführender Verbindung stehenden ersten Fördermittel (8, 10, 12),
   einem mit dem Behälter durch eine zweite Flüssigkeitsleitung (17) in fluidführender Verbindung stehenden zweiten Fördermittel (18),

einer Steuer- und Recheneinheit (110) konfiguriert zum

Ansteuern des ersten Fördermittels (8, 10, 12)

zum Hineinfördern eines ersten Flüssigkeitsvolumens in den zumindest teilweise mit Luft gefüllten Behälter (6)

**oder**

zum Herausfördern eines ersten Flüssigkeitsvolumens aus dem zumindest teilweise mit Luft und zumindest teilweise mit Flüssigkeit gefüllten Behälter (6),

Ansteuern des zweiten Fördermittels (18)

zum Herausfördern eines zweiten Flüssigkeitsvolumens aus dem durch das erste Fördermittel (8, 10, 12) mit einem ersten Flüssigkeitsvolumen befüllten Behälter (6)

**oder**

zum Hineinfördern eines zweiten Flüssigkeitsvolumens in den durch das ersten Fördermittel (8, 10, 12) um einen erstes Flüssigkeitsvolumen entleerten Behälter (6),

Messen des Drucks in dem Behälter (6) mit einer ersten Druckmessung vor dem Starten der Förderung des ersten Flüssigkeitsvolumens,

Messen des Drucks in dem Behälter (6) mit einer zweiten Druckmessung nach dem Stoppen der Förderung des ersten Flüssigkeitsvolumens,

Messen des Drucks in dem Behälter (6) mit einer dritten Druckmessung nach dem Stoppen der Förderung des zweiten Flüssigkeitsvolumens,

Auswerten mindestens der Messwerte der ersten Druckmessung und der dritten Druckmessung als Maß zum Überprüfen der Förderleistung des ersten Fördermittels (8, 10, 12) und/oder des zweiten Fördermittels (18).

2. Vorrichtung nach Anspruch 1 **dadurch gekennzeichnet, dass** die Steuer- und Recheneinheit (110) zusätzlich ausgebildet ist zum

Vergleichen des Messwerts der dritten Druckmessung mit einem oberen Grenzwert und/oder einem unteren Grenzwert und Schließen auf eine unzulässige Abweichung der Förderleistung mindestens des ersten (8, 10, 12) und/oder des zweiten Fördermittels (18) bei Unterscheiten des unteren Grenzwerts oder Überschreiten des oberen Grenzwerts.

3. Vorrichtung nach Anspruch 1 oder 2 **dadurch gekennzeichnet, dass** die Steuer- und Recheneinheit (110) zusätzlich ausgebildet ist zum

Berechnen der Druckdifferenz aus dem Messwert der ersten Druckmessung und dem Messwert der dritten Druckmessung als Maß für die Abweichung der Förderleistung des zweiten Fördermittels (18) von der Förderleistung des ersten Fördermittels (8, 10, 12).

4. Vorrichtung nach Anspruch 3 **dadurch gekennzeichnet, dass** die Steuer- und Recheneinheit (110) zusätzlich ausgebildet ist zum

Vergleichen der Druckdifferenz mit einem oberen Grenzwert und/oder einem unteren Grenzwert und Schließen auf eine unzulässige Abweichung der Förderleistung des ersten (8, 10, 12) und/oder des zweiten Fördermittels (18) bei Unterscheiten des unteren Grenzwerts oder Überschreiten des oberen Grenzwerts.

5. Vorrichtung nach Anspruch 3 oder 4 **dadurch gekennzeichnet, dass** die Steuer- und Recheneinheit (110) zusätzlich ausgebildet ist zum

Berechnen einer Volumendifferenz als Produkt der Systemcompliance und der Druckdifferenz aus dem Messwert der ersten Druckmessung und dem Messwert der dritten Druckmessung.

6. Vorrichtung nach Anspruch 5 **dadurch gekennzeichnet, dass** die Steuer- und Recheneinheit (110) zusätzlich ausgebildet ist zum

Berechnen eines Volumenfehlers aus der Volumendifferenz abzüglich der Sollwertdifferenz aus dem Sollwert des ersten Fördervolumens und dem Sollwert des zweiten Fördervolumens.

7. Vorrichtung nach Anspruch 6 **dadurch gekennzeichnet, dass** die Steuer- und Recheneinheit (110) zusätzlich ausgebildet ist zum

Vergleichen des Volumenfehlers mit einem vorgegebenen unteren Grenzwert für den Volumenfehler und/oder einem vorgegebenen oberen Grenzwert für den Volumenfehler und Schließen auf eine unzulässige Abweichung der Förderleistung mindestens des ersten (8, 10, 12) und/oder des zweiten Fördermittels (18) bei Unterscheiten des unteren Grenzwerts oder Überschreiten des oberen Grenzwerts.

8. Vorrichtung nach einem der Ansprüche 2, 4 oder 7 **dadurch gekennzeichnet, dass** die Steuer- und Recheneinheit (110) zusätzlich ausgebildet ist zum Ausgeben eines akustischen und/oder optischen Alarms bei Unterschreiten des unteren Grenzwerts oder Überschreiten des oberen Grenzwerts.

9. Vorrichtung nach einem der Ansprüche 2, 4, 7 oder 8 **dadurch gekennzeichnet, dass** die Steuer- und Recheneinheit (110) zusätzlich ausgebildet ist zur
Durchführung der Korrektur der Förderleistung mindestens des ersten (8, 10, 12) oder des zweiten Fördermittels (18).

10. Vorrichtung nach einem der Ansprüche 1 bis 9 **dadurch gekennzeichnet, dass** die Steuer- und Recheneinheit (110) zusätzlich ausgebildet ist zum
Vorgeben des ersten Fördervolumens an einen ersten Schrittmotor durch Vorgeben mindestens einer ersten Schrittanzahl und einem ersten Schrittwinkel
und/oder
Vorgeben des zweiten Fördervolumens an einen zweiten Schrittmotor durch Vorgeben mindestens einer zweiten Schrittanzahl und einem zweiten Schrittwinkel.

11. Vorrichtung nach einem der Ansprüche 1 bis 9 **dadurch gekennzeichnet, dass** die Steuer- und Recheneinheit (110) zusätzlich ausgebildet ist zum
Vorgeben des ersten Fördervolumens des ersten Fördermittels (8, 10, 12) durch Vorgeben einer ersten Förderzeit und/oder Vorgeben des zweiten Fördervolumens des zweiten Fördermittels durch eine zweite Förderzeit.

12. Vorrichtung nach einem der Ansprüche 1 bis 11 **dadurch gekennzeichnet, dass** das erste Fördermittel (8, 10, 12) eine erste Verdrängerpumpe ist und das zweite Fördermittel eine zweite Verdrängerpumpe ist.

13. Vorrichtung nach Anspruch 12 **dadurch gekennzeichnet, dass** die erste Verdrängerpumpe und/oder die zweite Verdrängerpumpe aus der Gruppe Membranpumpe, Zahnradpuinpe und Kolbenpumpe ausgewählt sind.

14. Vorrichtung nach einem der Ansprüche 1 bis 13 **dadurch gekennzeichnet, dass** der Behälter (6) eine Mischkammer in einer Flüssigkeitsleitung eines Dialysierflüssigkeitssystems ist und das erste Fördermittel (8, 10, 12) in der Flüssigkeitsleitung stromaufwärts der Mischkammer angeordnet ist und das zweite Fördermittel (18) in der Flüssigkeitsleitung stromabwärts der Mischkammer angeordnet ist.

15. Blutbehandlungsvorrichtung aufweisend ein Dialysierflüssigkeitssystem (1) zur Bereitstellung einer Dialysierflüssigkeit und eine Vorrichtung gemäß einem der Ansprüche 1 bis 14.

**Claims**

1. A device for checking the delivery capacity of at least at one first delivery means (8, 10, 12) and/or one second delivery means (18) of an apparatus for extracorporeal blood treatment, having
a closed container (6), filled at least partially with air,
or
a closed container (6), filled at least partially with air and at least partially with liquid,
at least one agent for measuring the pressure (13) in the container (6),
a first delivery means (8, 10, 12), which is in fluid-carrying connection to the container (6) through a first fluid line (7),
a second delivery means (18), which is in fluid-carrying connection with the container through a second fluid line (17),
a control and processing unit (110), configured for
activating the first delivery means (8, 10, 12) for delivering a first liquid volume into the container (6) filled at least partially with air,
or
for delivering a first liquid volume out of the container (6), which is at least partially filled with liquid,
activating the second delivery means (18) for delivering a second liquid volume out of the container (6) filled with a first liquid volume by the first delivery means (8, 10, 12),
or
for delivering a second liquid volume into the container (6) which has been emptied of a first liquid volume by the first delivery means (8, 10, 12),
measuring the pressure in the container (6) with a first pressure measurement before starting the delivery of the first liquid volume,
measuring the pressure in the container (6) with a second pressure measurement after stopping the delivery of the first liquid volume,
measuring the pressure in the container (6) with a third pressure measurement after stopping the delivery of the second liquid volume,
analyzing the measured values of the first pressure measurement and the third pressure measurement as a measure for checking the delivery capacity of the first delivery means (8, 10, 12) and/or of the second delivery means (18).

2. The device according to claim 1, **characterized in that** the control and processing unit (110) is additionally designed for
comparing the measured value of the third pressure measurement with an upper limit value and/or a lower limit value and deducing an inadmissible deviation in the delivery capacity of at least one of the first delivery means (8, 10, 12) and/or second delivery means (18) when the result falls below the lower limit value or exceeds the upper limit value.

3. The device according to claim 1 or 2, **characterized in that** the control and processing unit (110) is additionally designed for calculating the pressure difference of the measured value of the first pressure measurement and the measured value of the third pressure measurement as a measure of the deviation in the delivery capacity of the second delivery means (18) from the delivery capacity of the first delivery means (8, 10, 12).

4. The device according to claim 3, **characterized in that** the control and processing unit (110) is additionally designed for
comparing the pressure difference with an upper limit value and/or a lower limit value and deducing an inadmissible deviation in the delivery capacity of the first delivery means (8, 10, 12) and/or the second delivery means (18) when the result falls below the lower limit value or exceeds the upper limit value.

5. The device according to claim 3 or 4, **characterized in that** the control and processing unit (110) is additionally designed for calculating a volume difference as a product of the system compliance and the pressure difference between the measured value of the first pressure measurement and the measured value of the third pressure measurement.

6. The device according to claim 5, **characterized in that** the control and processing unit (110) is additionally designed for
calculating a volume error from the volume difference minus the setpoint difference from the setpoint value of the first delivery volume and the setpoint value of the second delivery volume.

7. The device according to claim 6, **characterized in that** control and processing unit (110) is additionally designed for comparing the volume error with a preselected lower limit value for the volume error and/or a preselected upper limit value for the volume error and deducing an inadmissible deviation in the delivery capacity of at least the first delivery means (8, 10, 12) and/or the second delivery means (18) when the result falls below the lower limit value or exceeds the upper limit value.

8. The device according to any one of claims 2, 4 or 7, **characterized in that** the control and processing unit (110) is additionally designed for
output of an acoustic and/or optical alarm when the result falls below the lower limit value or exceeds the upper limit value.

9. The device according to any one of claims 2, 4, 7 or 8, **characterized in that** the control and processing unit (110) is additionally designed for
carrying out the correction of the delivery capacity of at least the first delivery means (8, 10, 12) or of the second delivery means (18).

10. The device according to any one of claims 1 to 9, **characterized in that** the control and processing unit (110) is additionally formed for
preselecting the first delivery volume on a first stepping motor by preselecting at least one first number of steps and one first stepping angle,
and/or
preselecting the second delivery volume on a second stepping motor by preselecting at least one second number of steps and one second stepping angle.

**11.** The device according to any one of claims 1 to 9, **characterized in that** the control and processing unit (110) is additionally formed for
preselecting the first delivery volume of the first delivery means (8, 10, 12) by preselecting a first delivery time and/or preselecting the second delivery volume of the second delivery means by a second delivery time.

**12.** The device according to any one of claims 1 to 11, **characterized in that** the first delivery means (8, 10, 12) is a first displacement pump and the second delivery means is a second displacement pump.

**13.** The device according to claim 12, **characterized in that** the first displacement pump and/or the second displacement pump is/are selected from the group of diaphragm pumps, gearwheel pumps and piston pumps.

**14.** The device according to any one of claims 1 to 13, **characterized in that** the container (6) is a mixing chamber in a fluid line of a dialysis fluid system, and the first delivery means (8, 10, 12) is arranged upstream from the mixing chamber in the liquid line, and the second delivery means (18) is arranged downstream from the mixing chamber in the liquid line.

**15.** A blood treatment device having a dialysis fluid system (1) for supplying a dialysis fluid and a device according to any one of claims 1 to 14.

**Revendications**

**1.** Dispositif de vérification de la capacité de convoyage d'au moins un premier (8, 10, 12) et/ou un second moyen de convoyage (18) d'un dispositif de traitement sanguin extracorporel comportant
un réservoir fermé rempli au moins partiellement d'air (6)
ou
un réservoir fermé rempli au moins partiellement d'air et au moins partiellement de liquide (6),
au moins un moyen de mesure de pression (13) dans le réservoir (6),
un premier moyen de convoyage (8, 10, 12) en liaison de conduction de fluide avec le réservoir (6) via une première conduite de liquide (7),
un second moyen de convoyage (18) en liaison de conduction de fluide avec le réservoir via une seconde conduite de liquide (17),
une unité de commande et de calcul (110) conçue pour
commander le premier moyen de convoyage (8, 10, 12)
pour faire rentrer un premier volume de liquide dans le réservoir (6) au moins rempli partiellement d'air
ou
pour faire sortir un premier volume de liquide du réservoir (6) au moins rempli partiellement d'air et au moins rempli partiellement de liquide,
commander le second moyen de convoyage (18)
pour faire sortir un second volume de liquide du réservoir (6) rempli d'un premier volume de liquide par le premier moyen de convoyage (8, 10, 12)
ou
pour faire rentrer un second volume de liquide dans le réservoir (6) vidé d'un premier volume de liquide par le premier moyen de convoyage (8, 10, 12),
mesure de la pression dans le réservoir (6) par une première mesure de pression avant de démarrer le convoyage du premier volume de liquide,
mesure de la pression dans le réservoir (6) par une deuxième mesure de pression après l'arrêt du convoyage du premier volume de liquide,
mesure de la pression dans le réservoir (6) par une troisième mesure de pression après l'arrêt du convoyage du second volume de liquide,
exploitation au moins des valeurs mesurées lors de la première mesure de pression et de la troisième mesure de pression en tant qu'indicateur pour la vérification de la capacité de convoyage du premier moyen de convoyage (8 10, 12) et/ou du second moyen de convoyage (18).

**2.** Dispositif selon la revendication 1, **caractérisé en ce que** l'unité de commande et de calcul (110) est de plus conçue pour
comparer la valeur mesurée lors de la troisième mesure de pression à une valeur limite supérieure et/ou une valeur limite inférieure et conclure à un écart inadmissible de la capacité de convoyage au moins du premier (8, 10, 12) et/ou du second moyen de convoyage (18) en cas de non-atteinte de la valeur limite inférieure ou de dépassement de la valeur limite supérieure.

3. Dispositif selon la revendication 1 ou 2, **caractérisé en ce que** l'unité de commande et de calcul (110) est de plus conçue pour
calculer la différence de pression à partir de la valeur mesurée lors de la première mesure de pression et de la valeur mesurée lors de la troisième mesure de pression en tant qu'indicateur d'écart entre la capacité de convoyage du second moyen de convoyage (18) et la capacité de convoyage du premier moyen de convoyage (8, 10, 12).

4. Dispositif selon la revendication 3, **caractérisé en ce que** l'unité de commande et de calcul (110) est de plus conçue pour
comparer la différence de pression à une valeur limite supérieure et/ou une valeur limite inférieure et conclure à un écart inadmissible de la capacité de convoyage du premier (8, 10, 12) et/ou du second moyen de convoyage (18) en cas de non-atteinte de la valeur limite inférieure ou de dépassement de la valeur limite supérieure.

5. Dispositif selon la revendication 3 ou 4, **caractérisé en ce que** l'unité de commande et de calcul (110) est de plus conçue pour
calculer une différence de volume en tant que produit de la conformité du système et de la différence de pression à partir de la valeur mesurée lors de la première mesure de pression et de la valeur mesurée lors de la troisième mesure de pression.

6. Dispositif selon la revendication 5, **caractérisé en ce que** l'unité de commande et de calcul (110) est de plus conçue pour
calculer une erreur de volume à partir de la différence de volume en soustrayant la différence de valeur théorique à partir de la valeur théorique du premier volume de convoyage et de la valeur théorique du second volume de convoyage.

7. Dispositif selon la revendication 6, **caractérisé en ce que** l'unité de commande et de calcul (110) est de plus conçue pour
comparer l'erreur de volume à une valeur limite inférieure prédéfinie pour l'erreur de volume et/ou une valeur limite supérieure prédéfinie pour l'erreur de volume et conclure à un écart inadmissible de la capacité de convoyage au moins du premier (8, 10, 12) et/ou du second moyen de convoyage (18) en cas de non-atteinte de la valeur limite inférieure ou de dépassement de la valeur limite supérieure.

8. Dispositif selon une des revendications 2, 4 ou 7 **caractérisé en ce que** l'unité de commande et de calcul (110) est de plus conçue pour
émettre une alarme acoustique et/ou optique en cas de non-atteinte de la valeur limite inférieure ou de dépassement de la valeur limite supérieure.

9. Dispositif selon une des revendications 2, 4, 7 ou 8 **caractérisé en ce que** l'unité de commande et de calcul (110) est de plus conçue pour
effectuer la correction de la capacité de convoyage au moins du premier (8, 10, 12) ou du second moyen de convoyage (18).

10. Dispositif selon une des revendications 1 à 9, **caractérisé en ce que** l'unité de commande et de calcul (110) est de plus conçue pour
préconiser le premier volume de convoyage à un premier moteur pas à pas en préconisant au moins un premier nombre de pas et un premier angle de pas
et/ou
préconiser le second volume de convoyage à un second moteur pas à pas en préconisant au moins un second nombre de pas et un second angle de pas.

11. Dispositif selon une des revendications 1 à 9, **caractérisé en ce que** l'unité de commande et de calcul (110) est de plus conçue pour préconiser le premier volume de convoyage du premier moyen de convoyage (8, 10, 12) en préconisant une première durée de convoyage et/ou en préconisant le second volume de convoyage du second moyen de convoyage par une seconde durée de convoyage.

12. Dispositif selon une des revendications 1 à 11, **caractérisé en ce que** le premier moyen de convoyage (8, 10, 12) est une première pompe de refoulement et le second moyen de convoyage est une seconde pompe de refoulement.

13. Dispositif selon la revendication 12, **caractérisé en ce que** la première pompe de refoulement et/ou la seconde pompe de refoulement sont sélectionnées dans le groupe suivant : pompe à membrane, pompe à roue crantée et pompe à piston.

14. Dispositif selon une des revendications 1 à 13, **caractérisé en ce que** le réservoir (6) est une chambre de mélange dans une conduite de liquide d'un système de liquide de dialyse et que le premier moyen de convoyage (8, 10, 12) est disposé dans la conduite de liquide en amont de la chambre de mélange et le second moyen de convoyage (18) dans la conduite de liquide en aval de la chambre de mélange.

15. Dispositif de traitement sanguin présentant un système de liquide de dialyse (1) pour fournir un liquide de dialyse et un dispositif selon une des revendications 1 à 14.

**Figur 1**

EP 2 717 939 B1

Figur 2

Figur 3

Figur 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2009012455 A